Europäisches Patentamt

European Patent Office

Office européen des brevets

(19)

(11) Publication number: **0 620 972 A1**

(12) **EUROPEAN PATENT APPLICATION**

(21) Application number: 94109649.7

(22) Date of filing: 10.09.90

(51) Int. Cl.5: **A01N 63/00**, C12N 1/20,
//(C12N1/20,C12R1:38)

(30) Priority: 11.09.89 US 405138
06.09.90 US 601653

(43) Date of publication of application:
26.10.94 Bulletin 94/43

(60) Publication number of the earlier application in accordance with Art.76 EPC: **0 494 261**

(84) Designated Contracting States:
AT BE CH DE DK ES FR GB IT LI LU NL SE

(71) Applicant: **CROP GENETICS INTERNATIONAL CORPORATION**
**10150 Old Columbia Road**
**Columbia, MD 21046-1704 (US)**

(72) Inventor: **Carlson, Peter S.**
**900 Clifton Drive**
**Alexandria, Virgina 22308 (US)**

(74) Representative: **Patentanwälte Grünecker, Kinkeldey, Stockmair & Partner**
**Maximilianstrasse 58**
**D-80538 München (DE)**

(54) Microorganism strain of pseudomonas syringae.

(57) The invention relates to a microorganism strain of Pseudomonas syringae, a composition comprising cells of said microorganism strain and an adjuvant and/or medium and the use of said microorganism strain as a bioherbicide.

EP 0 620 972 A1

CROSS-REFERENCE TO RELATED APPLICATION

This application is a continuation-in-part application of co-pending U.S. Patent Application Serial No. 07/405,138, filed September 11, 1989.

Technical Field

Background Art

The present invention relates to a biological method for controlling the growth of unwanted plants by treatment with both a chemical agent, such as a herbicide, and a bacterial plant pathogen.

Widespread use of chemical herbicides has created concern about possible adverse ecological effects caused by large quantities of chemical agents that are being introduced into the environment. There are fears that these chemicals might pose risks to human and animal health. Special concern has been directed to health effects on agricultural workers, who may be exposed to high levels of chemical herbicides which may affect their health. In fact, it has been necessary to withdraw or severely restrict usage of some chemical herbicides from the market after adverse environmental or health effects became evident. Disposal of empty containers for chemical herbicides is heavily regulated, and the proper disposal of these containers can be inconvenient and expensive for farmers. Disposal sites for these containers are limited and illegal container disposal is a growing concern. There is a need, therefore, to find ways to decrease the quantity of chemicals that are used to control weeds and other unwanted vegetation.

One strategy for reducing the amount of chemical herbicides introduced into the environment is to use biological microorganisms as bioherbicides to kill weeds or inhibit their growth. Since the large majority of the known plant pathogens are fungi, research and commercial application involving bioherbicides has focused on the use of such mycoherbicidal fungi. For example, U.S. Patent No. 3,999,973 to Templeton teaches the use of Colletotrichum malvarum as a mycoherbicide for controlling prickly sida (Sida spinosa L.). U.S. Patent No. 4,606,751 to Van Dyke et al. describes the use of Bipolaris sorghicola as a mycoherbicide for controlling the growth of Johnsongrass (Sorghum haepense). U.S. Patent No. 4,636,386 to Anderson et al. discloses the use of a strain of Alternaria zinniae for use in controlling the growth of Italian thistle (Carduus pycnocephalus). Fusarium roseum Culmorum may be used for the mycoherbicidal control of Hydrilla verticillata (U.S. Patent No. 4,263,036 to Charudattan). For further discussion of the use of mycoherbicides, see R. Charudattan, "The Use of Natural and Genetically Altered Strains of Pathogens for Weed Control" in M.A. Hoy and D.C. Herzog (eds.), Biological Control in Agricultural IPM Systems, N.Y.: Academic Press, 1985, pp. 347-372 and G.E. Templeton, "Biological Control of Weeds," Amer. J. Alternative Agr. 3: 69-72 (1989).

The fungal plant pathogens that have been developed as mycoherbicides are obligate pathogens which infect only a single species or narrow range of plant host species. Fungal phytopathogens have been used as mycoherbicides where control of specific plant species is desired. Fungi have not been developed as broad range mycoherbicides for the control of multiple weed species. An additional disadvantage of fungal plant pathogens used as mycoherbicides is that, compared to phytopathogenic bacteria, they are relatively more expensive and difficult to produce and to keep viable on a commercial, large scale basis.

In some instances, chemical herbicides may be used in combination with a mycoherbicide to enhance control of weeds that are tolerant to the chemical herbicide or the mycoherbicide. E.g., R.A. Klerk et al., Integration of a Microbial Herbicide into Weed and Pest Control Programs in Rice (Oryza sativa), Weed Sci. 33: 95-99 (1985); U.S. Patent No. 4,755,208 to Riley and Walker. Mycoherbicides have also been used in combination with chemical herbicides where both attack the same weeds. See, e.g., U.S. Patent No. 4,776,873 to Caulder et al. However, the chemical agents have not been reported to increase the host range of the mycoherbicidal fungi.

Although bacterial plant pathogens usually infect a narrow range of host species under normal conditions, they are often capable of infecting and damaging a broad range of plant hosts opportunistically if the plants are stressed. For example, Pseudomonas syringae pathovar (pv.) tabaci infects primarily tobacco plants (Nicotiana tabacum L.), causing wildfire disease of tobacco. However, Pseudomonas syringae pv. tabaci is capable of opportunistically infecting a broad range of plants including 23 plant genera. G.B. Lucas, Diseases of Tobacco, 3rd Ed., Raleigh, N.C.: Biological Consulting Associates, 1975, pp. 397-409. The range of plant species that can be opportunistically infected by Pseudomonas syringae pv. tabaci is increased when host plants are subjected to environmental stress. Id. Other species of phytopathogenic bacteria are also capable of opportunistically infecting and injuring stressed plants. D.F. Schoenweiss, "Predisposition, Stress and Plant Disease," Ann. Rev. Phytopathol. 13: 193-211 (1975).

However, the ability of phytopathogenic bacteria to infect a broad range of hosts has not found any practical application because methods for promoting controlled, reproducible infections have not been available and because research has focused on controlling single weed species not amenable to chemical herbicide control. Therefore, opportunistic phytopathogenic bacteria have not been used as bioherbicides.

Thus, there exists a need in the art for compositions and methods for controlling a broad range of weed species while decreasing the quantity of herbicidal chemicals used for this purpose.

Disclosure of the Invention

It has been discovered that chemical agents such as chemical herbicides can be used to stress plants in a way that promotes opportunistic infection by bacterial plant pathogens. This discovery makes it possible to combine chemical agents and bacterial plant pathogens to provide means for control of a broad range of weed species.

More particularly, the present invention overcomes the problems and disadvantages of the prior art by providing a method to control the growth of a broad spectrum of weeds while reducing the amount of chemical herbicides used to achieve this goal. Bacterial plant pathogens and chemical agents such as herbicides are applied to weeds such that the weeds are controlled to a greater degree than would be obtained with either the chemicals or the phytopathogenic bacteria alone.

Accordingly, an object of this invention is to provide a means to control the growth of weeds by combining chemical agents, such as herbicides, with bacteria that infect plants, thus achieving greater weed control than is obtained by the same amount of chemical alone. Another object is to control the growth of weeds while reducing the amount of chemical introduced into the environment, thus reducing the worker exposure, environmental burden and container disposal problems associated with the use of chemical herbicides. Another object is to provide a general method for bioherbicidal control of weed growth that is safer than current methods, inexpensive and effective. Another object is to provide a general method to expand the spectrum of weeds controlled by specific chemical herbicides. Additional objects and advantages of the invention will be set forth in part in the description which follows, and in part will be apparent from the description or may be learned by practice of the invention. The objects and advantages of the invention will be realized and attained by means of the methods and combinations particularly pointed out in the appended claims.

To achieve the objects of the invention, as embodied and broadly described herein, one embodiment of the invention comprises a method of controlling the growth of weeds by applying a chemical agent to the weeds and simultaneously or subsequently applying a bacterial plant pathogen to the weeds, wherein inhibition of weed growth or killing of the weeds is greater than would be obtained by the same amounts of chemical or bacteria applied alone. Another embodiment of the invention comprises a composition for use in weed control comprising bacteria capable of infecting stressed plants and a chemical agent that causes stress and thus facilitates infection.

The following general description is offered by way of explanation and illustration, and not by way of limitation.

Brief Description of the Drawings

Figure 1 shows the effect of dicamba alone 0.067 kg/ha (0.06 lb/A) (broken line) or dicamba plus Pseudomonas syringae pv. tabaci (solid line) on three weeds (velvetleaf, foxtail and barnyardgrass).

Figure 2 shows the effect of glyphosate alone 0.28 hg/ha (0.25 lb/A) (broken line) or glyphosate plus Pseudomonas syringae pv. tabaci (solid line) on morningglory.

Best Mode for Carrying Out the Invention

For purposes of this application, a weed is "any plant that is objectionable or interferes with the activities or welfare of man." A herbicide (here called a chemical herbicide) is a "chemical used to control, suppress, or kill plants, or to severely interrupt their normal growth processes." Herbicide Handbook of the Weed Society of America, Fifth Edition (1983), xxi-xxiv. As used herein, the term "bioherbicide" means a biological organism used to control, suppress, or kill plants, or to severely interrupt their normal growth processes. The term "mycoherbicide" means any fungal organism used to control, suppress or kill plants, or to severely interrupt their normal growth processes.

To practice the invention, a chemical agent that facilitates bacterial infection and a bacterial plant pathogen capable of opportunistic infection are both applied topically to weeds. The chemical agent and bacterial plant pathogen are applied to the weeds by well-known methods of application such as spraying or as a soil drench.

Application may be preemergent, postemergent or as a nonselective contact herbicide. In the preferred embodiments described below, the chemical agent and bacterial plant pathogen are applied to weeds. The invention can be used to control weeds in an agricultural setting and can also be used for nonagricultural applications. For example, the invention can be used for the control of weeds in turf and as a contact bioherbicide for the management of roadside vegetation.

The chemical and the phytopathogenic bacteria can be applied simultaneously. In other circumstances, it may be preferable to apply the chemical agent first, and to apply the phytopathogenic bacteria later after allowing time for the chemical to act upon the plant. Whether simultaneous or sequential application is optimal will depend on the particular combination of chemical agent and phytopathogenic bacteria selected. If the chemical and bacteria are applied simultaneously, they can be mixed in the same formulation and sprayed on the weeds from the same tank. Compositions for practice of the invention can be formulated in numerous ways, including flowables, dry flowables, water dispersible granules or emulsified concentrates. Mixtures of different species or pathovars of bacterial plant pathogens can be used to practice this invention. The invention can also be practiced by applying a mixture of different chemical agents in conjunction with the bacterial plant pathogen(s).

The formulation of the invention may also contain adjuvants such as surfactants and suspension agents. Effective infection by the phytopathogenic bacteria can be facilitated and enhanced by applying the bacteria suspended in a medium that sustains or promotes the growth of the bacteria. This helps the bacteria to survive and grow during the period immediately after application to the weeds, increasing the likelihood that the phytopathogenic bacteria will successfully infect the weeds.

Various chemical agents can be used to practice this invention. Any chemical agent is suitable if it (1) injures, stresses, or otherwise acts on weeds to facilitate opportunistic phytopathological bacterial infection, and (2) does not inhibit the phytopathogenic bacteria or otherwise interfere with its ability to opportunistically infect plants. Different types of chemical herbicides can be used as the chemical agent. Herbicides that act as plant hormone mimics or photosynthesis inhibitors can render plants susceptible to bacterial infection but would not be expected to damage or interfere with bacterial action. Herbicides that act as metabolic inhibitors can injure certain bacteria, but the invention can still be practiced by using phytopathological bacterial strains that are resistant to or tolerant to or acclimated to the metabolic inhibitor. If the herbicidal chemical is applied separately from the phytopathological bacteria, then agents potentially injurious to the bacteria can be employed. The preferred embodiments described below employed glyphosate, sulfosate and dicamba as chemical agents. The invention has also been practiced using atrazine and 2,4-dichlorophenoxyacetic acid (2,4-D). For other chemical herbicides that can be used to practice this invention, see Herbicide Handbook of the Weed Society of America, Fifth Ed. (1983) and H. J. Lorenzini and L.S. Jeffrey, Weeds of the United States and Their Control, N.Y.: Van Nostrand, 1987, Table 1.9.

While such chemical herbicides can be used to practice this invention, it should be appreciated that chemicals which do not fit the conventional definition of herbicide could also be used. A chemical agent may stress a plant sufficiently to facilitate an opportunistic bacterial infection even though the chemical alone does not kill plants or severely interrupt normal growth processes. For example, plant hormone analogues that are not useful alone as herbicides may nevertheless facilitate bacterial infection.

The invention here described can be practiced using naturally occurring bacterial plant pathogens that are readily obtained from public depositories or isolated from the soil, plants, air, water, insects, etc., using techniques well known in the art. Pseudomonas syringae pv. tabaci has been found to be useful for this purpose, but other species of bacterial pathogens can be used. For example, Pseudomonas solanacearum has been used successfully to practice this invention. Other phytopathogenic bacteria my be selected from several prokaryotic genera including, but not limited to, Pseudomonas, Xanthomonas, Agrobacterium, Erwinia, Corynebacterium, Streptomyces, and Nocardia. See N.W. Schaad, How phytopathogenic bacteria are classified, in M.S. Mount and G.H. Lacy (eds.), Phytopathogenic Prokaryotes, Vol. 1, N.Y., Academic Press, 1982, pp. 19-29. Furthermore, wellknown techniques for the isolation or selection of mutants, either naturally occurring or induced, can be used to obtain bacteria with additional desirable features. Techniques of genetic engineering and recombinant DNA can also be used to produce bacteria with desirable characteristics.

Because phytopathogenic bacteria occur ubiquitously in nature, spraying these bacterial species in the field is not expected or anticipated to cause ecological problems. However, if it is deemed desirable to reduce the persistence of the bacteria, several strategies can be used. For example, temperature-sensitive bacteria which will not survive when environmental temperatures change can be selected as mutants or produced by genetic engineering using well-known techniques. Thus, cold-sensitive bacterial pathogens can be used as bioherbicides during the spring or summer, but will not survive the cold of winter. Similarly, bacteria sensitive to high temperatures can be used as bioherbicidal agents during the spring that will die in the higher temperatures of summer.

Another approach is to select auxotrophic mutants of phytopathogenic bacteria which require an essential nutrient that is available in infected plants, but is not present in sufficient quantities in the environment. Such nutrients may include amino acids, nucleic acid bases, or vitamins. Such phytopathogenic bacteria will act in living weeds, but cannot survive if they fail to infect a weed or after an infected weed dies. Another method to limit the persistence of the bacteria is to use recombinant DNA techniques to produce bacteria containing "suicide genes" that limit the persistence of the bacteria or allow only limited bacterial proliferation after application. See R. Curtiss III, Engineering organisms for safety: what is necessary?, in M. Sussman et al. (eds.), The Release of Genetically-engineered Microorganisms, N.Y., Academic Press, 1988, pp. 10-11.

An advantage of this method is that, unlike the phytopathogenic fungi used as mycoherbicides, bacterial plant pathogens can more easily and economically be grown in large quantities using well-known fermentation techniques.

It should be understood that in practicing this broad approach to weed control, each particular chemical agent and particular bacterial pathogen at specific dosages will not necessarily be effective. This is consistent with the behavior of other broad range chemical herbicides, which are rarely, if ever, effective in controlling all types of weeds and are usually ineffective in controlling some weed species. Whether a particular combination of herbicide and bacteria will be useful for a particular application, and the optimal conditions for the particular combination, can readily be determined by those skilled in the art without undue experimentation by simple testing in the greenhouse or the field. Certain principles may guide the selection of parameters. First, the effect of the phytopathogenic bacteria is most evident when the herbicide is applied at a level where the herbicide alone produces weak or marginal effects on the weeds. Where the herbicide at a particular dosage already produces a high degree of weed control, additional benefits from the addition of phytopathogenic bacteria may not be apparent. Bioherbicidal control is more apparent when the weed shows partial response to the level of chemical herbicide employed. Preferred levels for the application of three herbicides, glyphosate, dicamba, and sulfosate are illustrated in Examples 3 through 6 below, and effects of varying the concentration of a chemical herbicide are shown in Example 7. Second, greater effects are seen when greater quantities of bacteria are applied. In preferred embodiments, phytopathogenic bacteria mixed with the chemical are applied at a concentration of at least $5 \times 10^5$ CFU/ml, and preferably at $10^6$ CFU/ml or greater. In more preferred embodiments, the concentration of bacteria is $10^8$ CFU/ml.

Third, it will be apparent to those skilled in the art that while the rate of application of the chemical can advantageously be less than the usual field application rate (e.g., 25-50% in Examples 3 and 4; 12.5% in Examples 5 and 6), some level of the chemical will be too low to have a useful effect. Thus, dicamba and glyphosate are applied at about one-half to one-quarter of the usual field application levels in the currently preferred embodiments (see Examples 3 and 4), and sulfosate is applied at about one-eighth of the recommended field application level in the currently preferred embodiments (see Examples 5 through 7). Further reductions may be possible for particular applications.

Since the applied phytopathogenic bacteria infect plants stressed by the chemical agent, the spectrum of plant hosts infected by the phytopathogenic bacteria generally expands to resemble the spectrum of activity of the chemical herbicide. Sometimes, however, the spectrum of plants controlled by the chemical herbicide may be expanded. Certain weed species may not be controlled by a particular herbicide because the field application rate is too low. However, actions of the herbicide that are not otherwise manifested may be sufficient to facilitate opportunistic infection. Thus, combining a broad range chemical herbicide with a bacterial plant pathogen may serve not only to control the same spectrum of weeds with a lower level of chemical herbicide, but may also permit the control of weed species that are not usually controlled by the chemical herbicide (e.g., control of grasses with dicamba - see Example 3).

Practice of this invention may make it possible to achieve further reductions in the amount of chemical herbicides employed by giving users the option to replace the traditional prophylactic preemergent herbicide application with more efficient postemergent application. A postemergent chemical herbicide used in conjunction with a bacterial plant pathogen could be applied selectively only as required, providing an alternative to heavy preventative preemergent application of herbicides. This approach has not been generally feasible in the past due to the cost and limited spectrum of most postemergent herbicides.

Practice of this invention would have the additional advantage of reducing the costs associated with the disposal of chemical herbicide containers. This reduction would be accomplished by a reduction in the total amount of chemical herbicide used. In addition, the containers for the bacteria and for certain compositions combining bacteria with chemicals may be biodegradable.

The following examples are offered by way of illustration of the preferred embodiments and not by way of limitation, with the true scope of the invention being indicated by the claims which follow.

Example 1 illustrates a method for obtaining and growing preferred phytopathogenic bacteria that can be used to practice this invention.

EXAMPLE 1

Pseudomonas syringae pv. tabaci was obtained from the American Type Culture Collection (12301 Parklawn Drive, Rockville, Maryland 20852) (ATCC isolate #11527). This strain was used in Examples 1 through 4. The bacteria were grown in modified King's B medium (Table 1). The medium was supplemented with zinc, which enhances the production of toxin by these bacteria. R. D. Durbin and T. F. Uchytil, The Role of Zinc in Regulating Tabtoxin Production, Experientia 41: 136-37 (1985).

Table 1

| Modified King's B Medium with Zinc | |
|---|---|
| | (per liter) |
| Proteose peptone No. 3 (Difco*) | 20 g |
| Glycerol | 15 ml |
| $K_2HPO_4.3H_2O$ | 1.5 g |
| $MgSO_4.7H_2O$ | 1.5 g |
| Agar (Difco*) | 20 g |
| $ZnSO_4.7H_2O$ | .0075 g |
| pH 7.2 King, E.O., Ward, M.K., and Raney, D.E. Two Simple Media for the Demonstration of Pyocyanin and Fluorescin. J. Lab. Clin. Med. 44: 301-307 (1954). | |

*Difco Laboratories, Inc., Detroit, Michigan 48232. Bacteria were scraped from the culture plates using a rubber policeman and were suspended in water for mixture with chemical herbicides. Serial dilutions were spread-plated onto King's B medium to test for viability and for the absence of contaminants. Tobacco leaves sprayed with Pseudomonas syringae pv. tabaci developed yellow lesions and wilted, curled leaves demonstrating that the isolated strain retained virulence.

Example 2 illustrates testing the bacteria for compatibility with different chemical agents and describes methods for practicing the invention.

EXAMPLE 2

Herbicides tested were dicamba (3,6-dichloro-o-anisic acid) which was applied at 0.28-0.56 kg/ha (0.25 and 0.5 lb/A [pounds/acre]) and glyphosate (N-(phosphonomethyl)glycine) which was applied at .067-.140 kg/ha (0.06 and 0.125 lb/A). These application rates were chosen to be about 25% and 50% of the recommended field rates. Each experiment compared the effects of herbicide alone with the effects of herbicide mixed with Pseudomonas syringae pv. tabaci. The concentration of Pseudomonas syringae pv. tabaci in different experiments ranged from approximately $10^5$ to $10^6$ CFU/ml (colony forming units/ml), but all plants in each particular experiment received the same concentration of bacteria. In some experiments the mixture also contained CIDEKICK® (5.02 ml/L [4.75 ml/quart]), a surfactant, or ATTAGEL 40® (0.5%), a suspension agent, as adjuvants. CIDEKICK® is manufactured by JLB International Chemical, Inc. (P.O. Box 6006, Vero Beach, Florida 32961-6006) and ATTAGEL 40® is manufactured by Englehard Corporation (Specialty Chemicals Division, 33 Wood Avenue South, Menlo Park CN28, Edison, New Jersey 08818).

Preliminary experiments showed that Pseudomonas syringae pv. tabaci survived in dicamba and glyphosate at the concentrations employed in the tank mix for at least 120 minutes. Viability was also not significantly harmed during at least 120 minutes in CIDEKICK® (5.02 ml/L [4.75 ml/quart]) or ATTAGEL 40® (0.5%). On the other hand, Pseudomonas syringae pv. tabaci was killed within an hour when suspended in 0.719 g/L (0.006 lb/gal) of paraquat (1,1'-dimethyl-4,4'-bipyridium dichloride). Thus, paraquat could not be

EP 0 620 972 A1

used in a mixture with Pseudomonas syringae pv. tabaci.

The following weeds were tested: jimsonweed (Datura stramonium), velvetleaf (Abutilon theophrasti), morningglory (Ipomea purpurea), barnyardgrass (Echinochloa crusgalli), and yellow foxtail (Setaria glauca). These were selected as being weeds of economic importance in row crops and as representing a spectrum of plant families. Tobacco plants (Nicotiana tabacum L.) were used to test the virulence of the Pseudomonas syringae pv. tabaci. Seedlings were grown in 10.2 um (4 inch) pots in a greenhouse under ordinary greenhouse conditions. Plants were sprayed with their assigned treatment in an enclosed plexiglas chamber using a $CO_2$ pressurized spray unit fitted with a flood jet nozzle mounted on top of the chamber. The nozzle delivered approximately 234 L/ha (25 gallons/acre) at 1.3 bars (15 pounds per square inch). The experimental design was a randomized complete block design with 2 replications per treatment and 2 plants per replication. Plastic bags were placed over all plants treated with Pseudomonas syringae pv. tabaci for approximately one hour after application.

Injury was rated weekly on a scale of 0-100 with 0 = no injury and 100 = plant death. This type of rating evaluation is widely used to evaluate the effects of herbicides (e g., Eberlein et al., Weed Science 36: 792-799 (1988)).

In order to confirm that weeds were being infected by the phytopathological bacteria sprayed onto the plants with the herbicides, mutants of Pseudomonas syringae pv. tabaci were developed that were resistant to an antibiotic, rifampicin. Cultures of Pseudomonas syringae pv. tabaci (ATCC strains 11527 and 15373) were grown in medium containing 100 micrograms/ml of rifampicin. Resistant colonies were propagated for further study. Selected strains were inoculated into tobacco plants to demonstrate virulence. Strains of Pseudomonas syringae pv. tabaci were then reisolated from lesions in the tobacco plants and further propagated in medium containing 100 microliters/ml of rifampicin. One strain was selected for further testing on weeds in combination with chemical herbicides. Some enhanced weed control was seen from the combination of the mutant Pseudomonas syringae pv. tabaci and the chemical herbicides, although the bacterial mutant was less effective in enhancing weed control than the wild-type bacterial strain. Rifampicin-resistant Pseudomonas syringae pv. tabaci was isolated from infected weeds, demonstrating that the bacteria applied with the chemical herbicide was the source of the infection.

Examples 3 and 4 illustrate results of practicing the invention with two different chemical agents.

EXAMPLE 3

Five kinds of weeds (jimsonweed, velvetleaf, morningglory, barnyardgrass and yellow foxtail) were treated with 0.067 kg/ha (0.06 lb/A) of dicamba, with or without Pseudomonas syringae pv. tabaci. Pseudomonas syringae pv. tabaci was applied at approximately 5 X $10^5$ CFU/ml. CIDEKICK®, 5.02 ml/L (4.75 ml/quart), was included as an adjuvant. Temperature at the time of application was 82°F. The degree of injury was rated at 7, 14, 20 and 28 days. The results for velvetleaf, foxtail and barnyardgrass are shown in Figure 1. These three weeds showed significantly greater injury to the weeds when the bacteria and dicamba were applied together than when the dicamba was used alone. Jimsonweed and morningglory (not shown) were already controlled to a relatively high degree (60-100%) by dicamba alone, so there was little enhancement of injury from the inclusion of Pseudomonas syringae pv. tabaci.

The markedly enhanced control of barnyardgrass and foxtail is especially interesting because dicamba is usually most effective against broadleaf weeds, and is usually relatively ineffective on grasses. Thus, combining the bacterial pathogen with dicamba increased the range of species of weeds controlled by the chemical herbicide even though the amount of herbicide was lower than the amount recommended for use in the field. Recommended rates for dicamba in the field range from 0.28-8.97 kg/ha (0.25 to 8.0 lb/A).

EXAMPLE 4

Five kinds of weeds (jimsonweed, velvetleaf, morningglory, barnyardgrass and yellow foxtail) were sprayed with 28 kg/ha (0.25 lb/A) of glyphosate, with or without Pseudomonas syringae pv. tabaci. CIDEKICK®, 5.02 ml/L (4.75 ml/quart), was included as an adjuvant. Pseudomonas syringae pv. tabaci was applied at approximately 5 X $10^5$ CFU/ml. Temperature at the time of application was 27.8°C (82°F). The degree of injury was rated at 7, 14, 20 and 28 days.

Jimsonweed, velvetleaf, barnyardgrass and yellow foxtail were already controlled to a fairly high level (57.5-100%) by glyphosate alone, so control was not significantly different when Pseudomonas syringae pv. tabaci was added. However, morningglory was more resistant to 0.28 kg/ha (0.25 lb/A) of glyphosate alone. The injury to morningglory was enhanced when Pseudomonas syringae pv. tabaci was applied with the glyphosate (Figure 2). Thus, combining the bacterial pathogen with glyphosate increased the range of

7

species of weeds controlled by the herbicide even though the amount of herbicide was lower than the amount recommended for use in the field. Recommended rates for glyphosate in the field range from 0.56-4.48 hg/ha (0.5 to 4.0 lb/A).

EXAMPLE 5

The effects of Pseudomonas syringae pv. tabaci in combination with the herbicide sulfosate (trimethylsulfonium carboxymethylaminomethylphosphonate; described in U.S. Patent No. 4,315,765) obtained from ICI Agricultural Products, Wilmington, Delaware, was tested under greenhouse conditions. In Examples 5 through 7, the strain of Pseudomonas syringae pv. tabaci used (ATCC #55090), was isolated from a tobacco plant in Maryland by Dr. Arvydas Grybauskas of the Department of Botany, University of Maryland, College Park, Maryland. The effects were evaluated on nine kinds of weeds, including both broadleaf weeds (morningglory, velvetleaf, cocklebur [Xanthium strumarium], redroot pigweed [Amaranthus retroflexus] and jimsonweed) and grasses (barnyardgrass, yellow foxtail, johnsongrass and fall panicum [Panicum dichotomiflorum]). Four treatments were compared: water alone, sulfosate alone, Pseudomonas syringae pv. tabaci alone, or sulfosate plus Pseudomonas syringae pv. tabaci. Sulfosate was applied at 0.067 kg/ha (0.06 lb/A), which is one-eighth of the rate recommended for application in the field. The bacteria were applied at approximately $10^8$ CFU/ml.

All solutions (including the water control treatment) contained 0.25 % (v/v) of ORTHO X-77® as an adjuvant. X-77® is a spreading agent sold by Chevron Chemical Company. According to the manufacturer, active agents in X-77® are alkylarylpolyoxyethylene glycols, free fatty acids and isopropanol. Percent injury scores were determined as in the previous examples. The method for determining percent injury scores is described in Research Methods in Weed Science, 3rd Edition, Southern Weed Science Society, 1986, p. 37.

To standardize the treatments, all solutions were sprayed on the weeds using a Model SB8 Spray Booth manufactured by DeVries Manufacturing (Hollandale, Minnesota) that was purchased from R & D Sprayers, Inc. (Opelousas, Louisiana). This device permits highly reproducible spraying of the test solutions. The sprayer was set to deliver 280.8 L/ha (25 gallons/acre) with a nozzle pressure of 1.6-1.7 bars (23-25 pounds per square inch). The speed of the traveling spray nozzle was 4.83 km/h (3.0 miles per hour). Plants were sprayed individually in the chamber. Unlike Examples 3 and 4, the plants were not placed in plastic bags after spraying.

Two experiments were run. In the first experiment, the degree injury was rated at 3, 9 and 17 days after treatment. In the second experiment, the degree injury was rated at 4, 9 and 17 days after treatment. The experimental design was a randomized complete block design with 2 replications per treatment and at least 2 plants per replication. Injury ratings (Table 2) were expressed as averages of the replicate values. Jimsonweed was used only in the second experiment. The second experiment was run later in the spring, during which the weeds experienced higher average environmental temperatures.

The results of the experiments are shown in Table 2. As would be expected, spraying the weeds with water containing only X-77® adjuvant produced essentially no injury. The weeds varied in their susceptibility to the low levels of sulfosate used, with broadleaf weeds showing some injury (particularly in the second experiment) while there was little or no damage to the grasses. The bacteria alone (Table 2A) produced some injury to the broadleaf weeds at early times in the first experiment after which the plants recovered, and no injury to the grasses.

Weeds treated with the combination of sulfosate and Pseudomonas syringae pv. tabaci (Table 2A) generally showed a greater degree of injury than weeds treated with either treatment alone. Broadleaf weeds showed a substantial degree

8

## Table 2

### Effects of Sulfosate and *Pseudomonas syringae* pv. *tabaci* Alone and in Combination

#### Percent Plant Injury Scores[1]

| Treatment | DAT[2] | Morningglory | | Velvetleaf | | Cocklebur | | Pigweed | |
|---|---|---|---|---|---|---|---|---|---|
| | | 1st | 2d | 1st | 2d | 1st | 2d | 1st | 2d |
| H$_2$O | 3-4 | 0.0 | 5.0 | 0.0 | 0.0 | 0.0 | 0.0 | 0.0 | 0.0 |
| | 9 | 0.0 | 0.0 | 0.0 | 0.0 | 0.0 | 0.0 | 0.0 | 0.0 |
| | 17 | 0.0 | 0.0 | 0.0 | 0.0 | 0.0 | 0.0 | 0.0 | 0.0 |
| Sulfosate[3] | 3-4 | 0.0 | 22.5 | 22.5 | 32.5 | 40.0 | 47.5 | 20.0 | 7.5 |
| | 9 | 0.0 | 30.0 | 5.0 | 25.0 | 0.0 | 40.0 | 0.0 | 45.0 |
| | 17 | 0.0 | 27.5 | 0.0 | 0.0 | 0.0 | 52.5 | 0.0 | 20.0 |

#### A. Plate Cultures

| Treatment | DAT | Morningglory | | Velvetleaf | | Cocklebur | | Pigweed | |
|---|---|---|---|---|---|---|---|---|---|
| Bacteria[4] | 3-4 | 20.0 | 20.0 | 12.5 | 0.0 | 30.0 | 0.0 | 45.0 | 45.0 |
| | 9 | 25.0 | 30.0 | 0.0 | 0.0 | 0.0 | 0.0 | 0.0 | 25.0 |
| | 17 | 12.5 | 17.5 | 0.0 | 0.0 | 0.0 | 0.0 | 0.0 | 55.0 |
| Sulfosate[3] plus Bacteria[4] | 3-4 | 40.0 | 30.0 | 42.5 | 30.0 | 52.5 | 27.5 | 87.5 | 60.0 |
| | 9 | 70.0 | 47.5 | 45.0 | 22.5 | 47.5 | 30.0 | 100.0 | 60.0 |
| | 17 | 60.0 | 27.5 | 40.0 | 20.0 | 80.0 | 55.0 | 100.0 | 97.5 |

#### B. Liquid Cultures

| Treatment | DAT | Morningglory | | Velvetleaf | | Cocklebur | | Pigweed | |
|---|---|---|---|---|---|---|---|---|---|
| Bacteria[4] | 3-4 | 20.0 | 5.0 | 22.5 | 0.0 | 25.0 | 0.0 | 17.5 | 0.0 |
| | 9 | 25.0 | 20.0 | 0.0 | 0.0 | 27.5 | 0.0 | 0.0 | 12.5 |
| | 17 | 32.5 | 12.5 | 0.0 | 0.0 | 37.5 | 37.5 | 0.0 | 40.0 |
| Sulfosate[3] plus Bacteria[4] | 3-4 | 40.0 | 25.0 | 50.0 | 37.5 | 62.5 | 27.5 | 75.0 | 55.0 |
| | 9 | 60.0 | 27.5 | 45.0 | 30.0 | 55.0 | 25.0 | 97.5 | 67.5 |
| | 17 | 40.0 | 40.0 | 40.0 | 62.5 | 50.0 | 42.5 | 100.0 | 57.5 |

1. Percent plant injury scores determined as described in the text.
   0% = no effect; 100% = plant killed
2. DAT = Days After Treatment
3. Sulfosate applied at 0.067 kg/ha (0.06 lb/A) = 1/8 of recommended field rate
4. *Pseudomonas syringae* pv. *tabaci* applied at 10$^8$ CFU/ml; suspended in King's B Medium

## Table 2
### (Continued)

*Effects of Sulfosate and **Pseudomonas syringae** pv. **tabaci***
*Alone and in Combination*

Percent Plant Injury Scores[1]

| Treatment | DAT[2] | Jimsonweed | | Barnyardgrass | | Yellow Foxtail | | Fall Panicum | | Johnsongrass | |
|---|---|---|---|---|---|---|---|---|---|---|---|
| | | --- | 2d | 1st | 2d | 1st | 2d | 1st | 2d | 1st | 2d |
| H₂O | 3-4 | --- | 0.0 | 0.0 | 0.0 | 0.0 | 0.0 | 15.0 | 15.0 | 0.0 | 0.0 |
| | 9 | --- | 0.0 | 0.0 | 0.0 | 0.0 | 0.0 | 0.0 | 0.0 | 0.0 | 0.0 |
| | 17 | --- | 0.0 | 0.0 | 0.0 | 0.0 | 0.0 | 0.0 | 0.0 | 0.0 | 0.0 |
| Sulfosate[3] | 3-4 | --- | 20.0 | 0.0 | 0.0 | 0.0 | 5.0 | 5.0 | 5.0 | 0.0 | 7.5 |
| | 9 | --- | 32.5 | 0.0 | 0.0 | 0.0 | 20.0 | 0.0 | 0.0 | 0.0 | 0.0 |
| | 17 | --- | 25.0 | 0.0 | 0.0 | 0.0 | 0.0 | 0.0 | 0.0 | 0.0 | 0.0 |

**A.   Plate Cultures**

| Treatment | DAT[2] | Jimsonweed | | Barnyardgrass | | Yellow Foxtail | | Fall Panicum | | Johnsongrass | |
|---|---|---|---|---|---|---|---|---|---|---|---|
| Bacteria[4] | 3-4 | --- | 0.0 | 0.0 | 0.0 | 0.0 | 7.5 | 10.0 | 0.0 | 0.0 | --- |
| | 9 | --- | 0.0 | 0.0 | 0.0 | 0.0 | 65.0 | 0.0 | 0.0 | 0.0 | 0.0 |
| | 17 | --- | 0.0 | 0.0 | 0.0 | 0.0 | 32.5 | 0.0 | 0.0 | 0.0 | 0.0 |
| Sulfosate[3] | 3-4 | --- | 25.0 | 45.0 | 0.0 | 45.0 | 0.0 | 50.0 | 5.0 | 47.5 | 5.0 |
| plus | 9 | --- | 35.0 | 67.5 | 0.0 | 82.5 | 25.0 | 32.5 | 28.0 | 90.0 | 0.0 |
| Bacteria[4] | 17 | --- | 37.5 | 30.0 | 0.0 | 85.0 | 0.0 | 47.5 | 0.0 | 75.0 | 0.0 |

**B.   Liquid Cultures**

| Treatment | DAT[2] | Jimsonweed | | Barnyardgrass | | Yellow Foxtail | | Fall Panicum | | Johnsongrass | |
|---|---|---|---|---|---|---|---|---|---|---|---|
| Bacteria[4] | 3-4 | --- | 0.0 | 0.0 | 0.0 | 0.0 | 0.0 | 0.0 | 0.0 | 0.0 | 0.0 |
| | 9 | --- | 0.0 | 0.0 | 0.0 | 0.0 | 7.5 | 0.0 | 0.0 | 0.0 | 0.0 |
| | 17 | --- | 0.0 | 0.0 | 0.0 | 0.0 | 0.0 | 0.0 | 0.0 | 0.0 | 0.0 |
| Sulfosate[3] | 3-4 | --- | 22.5 | 37.5 | 10.0 | 40.0 | 7.5 | 50.0 | 12.5 | 42.5 | 15.0 |
| plus | 9 | --- | 35.0 | 55.0 | 47.5 | 75.0 | 65.0 | 25.0 | 57.5 | 77.5 | 17.5 |
| Bacteria[4] | 17 | --- | 37.5 | 35.0 | 12.5 | 77.5 | 32.5 | 42.5 | 20.0 | 85.0 | 7.5 |

1.   Percent plant injury scores determined as described in the text.
0% = no effect; 100% = plant killed
2.   DAT = Days After Treatment
3.   Sulfosate applied at 0.067 kg/ha (0.06 lb/A) = 1/8 of recommended field rate
4.   *Pseudomonas syringae* pv. *tabaci* applied at 10⁸ CFU/ml; suspended in King's B Medium

of injury in both experiments. Grasses treated with the combination showed substantial injury in the first experiment, although a lesser effect was found in the second experiment. Detailed differences between the first and second experiments may be related to environmental factors such as the time of year, temperature, or amount of light.

Example 6 demonstrates the use of bacteria grown in liquid culture.

EXAMPLE 6

Instead of growth of agar plates, Pseudomonas syringae pv. tabaci was grown in liquid cultures in King's B medium (Table 1) from which the agar was omitted. The cells were incubated in 2 liter Erlenmeyer flasks at 28°C. in a rotary shaker (New Brunswick G10 gyroshaker) at 180-190 r.p.m. for about 64 hours until the cells reached a concentration of approximately 10⁹ CFU/ml. The media containing the bacterial cells was diluted with fresh culture medium to a concentration of 10⁸ CFU/ml. After addition of X-77®, the resulting suspensions were used as additional treatment groups (Pseudomonas syringae pv. tabaci alone or

EP 0 620 972 A1

with sulfosate) in the experiments described in Example 5.

The results are shown in part B of Table 2. Some broadleaf weeds, particularly morningglory, were susceptible to injury by the Pseudomonas syringae pv. tabaci alone, but the grasses showed essentially no injury. However, all types of plants showed substantial injury after treatment with a combination of sulfosate and Pseudomonas syringae pv. tabaci, and the degree of injury was generally greater than that produced by either the sulfosate or bacteria alone. It is notable that although the grasses were all resistant to sulfosate or bacteria when applied individually, all showed substantial injury when the sulfosate and bacteria were applied together.

Example 7 illustrates the effect of varying the amounts of sulfosate applied.

EXAMPLE 7

Experiments were performed generally following the protocol of Example 6, but varying the amount of sulfosate applied. Sulfosate was applied at one-half, one-fourth, and one-twelfth the recommended field application rate of 0.54 kg/ha (0.48 lb/A) (i.e., at 0.27, 0.13, and 0.04 kg/ha [0.24, 0.12, and 0.04 lb/A] respectively). Pseudomonas syringae pv. tabaci grown in liquid cultures were applied at approximately $10^8$ CFU/ml.

Sulfosate at both one-half and one-fourth the recommended field rate produced significant injury. The injury ratings produced by sulfosate alone were generally over 40% for broadleaf weeds and 25% of greater for grasses. These results are consistent with the common observation that plants are usually more sensitive to herbicides under greenhouse conditions than they are to the same level of herbicide in the field.

The application of Pseudomonas syringae pv. tabaci in combination with one-fourth the recommended field rate of sulfosate generally increased the degree of injury. The degree of enhancement was less marked at one-fourth the recommended field rate than was seen when sulfosate was applied at one-eighth the recommended field rate (See Table 2B). For example, with 0.12 lb/A of sulfosate alone, morningglory showed 42.5% injury at 4 days and 47.5% injury at 17 days. With the addition of Pseudomonas syringae pv. tabaci, the injury ratings were 50.0% and 65.0%, respectively. Barnyardgrass showed 27.5% injury with sulfosate alone at one-fourth the recommended field rate at both time points. With the addition of the bacteria, the injury ratings were 30.0% at 4 days and 47.5% at 17 days. Other weeds showed higher degrees of injury with sulfosate alone, and in some cases there was no further increase in injury when Pseudomonas syringae pv. tabaci was added to the sulfosate.

Table 3 shows the results when sulfosate was applied at one-twelfth of the recommended field rate. At this level of application, only pigweed was highly sensitive to the sulfosate alone. The combination of sulfosate and Pseudomonas syringae pv. tabaci showed enhanced injury for all of the broadleaf weeds and for yellow foxtail.

These results, when compared to the results in Example 5, show that the addition of phytopathogenic bacteria to a chemical herbicide can produce enhanced injury to weeds over a range of herbicide concentrations. However, the combination of chemical herbicide and phytopathogenic bacteria is particularly effective when, as in Example 6, the level of herbicide is chosen to produce a measurable but low degree of injury when applied alone.

It will be apparent to those skilled in the art that various modifications and variations can be made in the practice

11

**Table 3**

*Effects of Sulfosate and Pseudomonas syringae pv. tabaci*
*From Liquid Cultures Alone and in Combination*

Percent Plant Injury Scores[1]

| Treatment | DAT[2] | Morningglory | Velvetleaf | Cocklebur | Pigweed | Barnyardgrass | Yellow Foxtail | Fall Panicum | Johnsongrass |
|---|---|---|---|---|---|---|---|---|---|
| H2O | 10 | 0.0 | 7.5 | 0.0 | 12.5 | 0.0 | 15.0 | 0.0 | 0.0 |
| H2O | 17 | 5.0 | 0.0 | 0.0 | 45.0 | 0.0 | 0.0 | 0.0 | 0.0 |
| Sulfosate[3] | 10 | 7.5 | 0.0 | 20.0 | 45.0 | 17.5 | 0.0 | 0.0 | 0.0 |
| Sulfosate[3] | 17 | 12.5 | 0.0 | 5.0 | 50.0 | 0.0 | 0.0 | 0.0 | 0.0 |
| Bacteria[4] | 10 | 30.0 | 0.0 | 0.0 | 0.0 | 12.5 | 0.0 | 0.0 | 7.5 |
| Bacteria[4] | 17 | 15.0 | 0.0 | 0.0 | 0.0 | 0.0 | 0.0 | 0.0 | 0.0 |
| Sulfosate[3] plus Bacteria[4] | 10 | 35.0 | 0.0 | 10.0 | 0.0 | 67.5 | 22.5 | 0.0 | 15.0 |
| Sulfosate[3] plus Bacteria[4] | 17 | 32.5 | 30.0 | 42.5 | 92.5 | 25.0 | 0.0 | 0.0 | 0.0 |

1. Percent plant injury scores determined as described in the text.
   0 % = no effect; 100 % = plant killed
2. DAT = Days After Treatment
3. Sulfosate applied at 0.04 kg/ha (0.04 lbs/acre) = 1/12 of recommended field rate
4. *Pseudomonas syringae* pv. *tabaci* - $10^8$ CFU/ml from liquid cultures; suspended in King's B Medium

of the present invention without departing from the scope or spirit of the invention. For example, the invention can be practiced using bacteria that have been genetically engineered to confer additional desirable characteristics. Other embodiments of the invention will be apparent to those skilled in the art from consideration of the specification and practice of the invention disclosed herein. Thus, it is intended that the present invention cover modifications and variations of this invention, provided they fall within the scope of the appended claims or their equivalents.

**Claims**

1.  Microorganism strain of Pseudomonas syringae deposited under accession number ATCC 55090, mutants and variants thereof having the same identifying characteristics as Pseudomonas syringae ATCC 55090.

2.  A composition comprising Pseudomonas syringae according to claim 1 and an adjuvant and/or medium.

3.  Use of Pseudomonas syringae according to claim 1 as a bioherbicide.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | R.GHERNA ET. AL. 'ATCC Catalogue of Bacteria and Phages' 1989 , ATCC , US * page 178 - page 182 * | 1-3 | A01N63/00 C12N1/20 //(C12N1/20, C12R1:38) |

TECHNICAL FIELDS SEARCHED (Int.Cl.5)

A01N
C12R

The present search report has been drawn up for all claims

| Place of search | Date of completion of the search | Examiner |
|---|---|---|
| THE HAGUE. | 26 July 1994 | Donovan, T |